# DEMANDE DE BREVET EUROPEEN

(11) **EP 4 825 422 A1**
(43) Date de publication de la demande: **21.10.2026**
(21) Numéro de dépôt: 26159674.6
(22) Date de dépôt: 19.02.2026
(51) Int. Cl.: A61M 16/00, A61M 16/12, A61M 16/10

(54) **APPAREIL DE DÉLIVRANCE DE NO AVEC LIGNE DE PRÉLÈVEMENT D AIR AMBIANT**

(30) Priorité: 18.04.2025 FR 2504173
(71) Demandeur: INOSYSTEMS, 92160 Antony (FR)
(72) Inventeur: PROUVEZ, Nathan, 92160 Antony (FR); REY MARTIN, Fernando, 92160 Antony (FR); SAAS, Axelle, 92160 Antony (FR); NOTIS, Nicolas, 92160 Antony (FR)
(74) Mandataire: Air Liquide

(57) **Abrégé**

L'invention concerne un appareil de délivrance de NO (1) pour véhiculer et injecter un mélange gazeux NO/azote dans un circuit patient (2) ; une ligne principale de prélèvement de gaz (4) ; une ligne d'analyse de gaz (5) mesurant les teneurs en NO et/ou en NO₂; des moyens d'aspiration de gaz (7) ; des moyens de pilotage (8) ; et des moyens d'affichage (9) de la teneur en NO et/ou en NO₂. Une ligne secondaire de prélèvement de gaz (20) communique avec l'atmosphère. Des moyens à vanne (21) raccordés aux lignes principale (4) et secondaire (20), et à la ligne d'analyse (5), sont pilotés par les moyens de pilotage (8) pour mettre la ligne d'analyse de gaz (5) en communication avec l'une ou l'autre des lignes de prélèvement de gaz (4, 20). Installation d'administration de gaz incluant l'appareil (1).

## Description

L'invention porte sur un dispositif ou appareil de délivrance de NO, c'est-à-dire un appareil de fourniture d'un gaz contenant du NO, destiné à être raccordé entre une (des) source de NO gazeux sous pression, telle une (des) bouteille de NO/N₂, et le circuit patient alimenté par un ventilateur médical, ledit appareil de délivrance de NO comprenant des moyens analyseur de gaz permettant de mesurer la teneur en NO et/ou NO₂ dans le flux de gaz final alimentant le patient ou, selon le cas, dans l'air ambiant, c'est-à-dire de l'air du lieu où se trouve l'appareil de délivrance de NO, telle une pièce, une salle, un véhicule ou analogue, et des moyens d'affichage pour afficher les valeurs correspondantes de NO et/ou NO₂; et une installation d'administration de gaz à un patient comprenant un tel appareil de délivrance de NO.

Le monoxyde d'azote ou NO est couramment utilisé pour traiter différentes conditions médicales, comme décrit notamment par EP-A-560928, EP-A-1516639 et US-A-10,201,564. En général, un mélange gazeux de NO (i.e. quelques ppm vol.) et d'azote (N₂) est injecté par un dispositif de délivrance de NO, dans un flux gazeux contenant de l'oxygène, tel un mélange N₂/O₂ ou de l'air, voire de l'oxygène pur, qui est véhiculé par le circuit patient d'une installation de fourniture de gaz, tel un ventilateur médical, fournissant le flux gazeux à base d'oxygène audit circuit patient et le mélange gazeux final (NO/N₂/O₂) ainsi obtenu contenant du NO, de l'oxygène et de l'azote est ensuite administré par inhalation au patient à traiter en ayant besoin, soit de manière invasive par une sonde trachéale, ou de manière non-invasive par masque ou analogue, comme décrit par US-A-5,558,083.

Pour s'assurer que le flux gazeux final NO/N₂/O₂ ne contient pas ou qu'une quantité négligeable d'espèces toxiques, i.e. de composés NO₂ résultant d'une oxydation intempestive d'une partie du NO par de l'O₂, l'appareil de délivrance de NO dispose d'une ligne de prélèvement de gaz permettant de prélever une petite quantité, i.e. un échantillon, du mélange gazeux final (NO/N₂/O₂) au sein du circuit respiratoire, comme décrit par US2006/207594 ou EP4537879. Ces échantillons gazeux sont ensuite analysés au sein d'une ligne d'analyse de l'appareil de délivrance de NO pour vérifier si la composition du flux gazeux final NO/N₂/O₂ correspond à celle souhaitée, notamment sa teneur en NO qui doit être inférieure à 80 ppmv, et surtout que sa teneur en NO₂ qui doit être la plus faible possible, typiquement moins de 5 ppmv, car le NO₂ est un gaz toxique et irritant pouvant entraîner de graves lésions chez le patient, même à faible concentration.

Le problème est que le personnel médical, i.e. médecins, infirmières ou autres, ou paramédical peut être exposé de plusieurs manières à ces gaz, potentiellement nocifs et irritants, i.e. NO et NO₂, au sein des établissements hospitaliers ou dans les véhicules de transport car ces gaz se retrouvent dans l'air ambiant des lieux ou locaux clos où sont utilisées les installations de traitement par NO, tels les pièces, les salles d'opération ou de réanimation hospitalières, les couloirs ou autres, mais aussi dans l'habitable des véhicules de transport, en particulier les véhicules d'urgence, tels que ambulances, hélicoptères, avions...

Ces composés NO et NO₂ s'accumulent dans l'air ambiant autour de l'installation de fourniture de NO en différentes circonstances et le personnel soignant peut être amené à les inhaler de manière intempestive et/ou inévitable, c'est-à-dire de manière non-volontaire.

Ainsi, en cas de purge des flexibles reliant les bouteilles de NO/N₂ au dispositif de délivrance de NO, typiquement lors d'un remplacement d'une bouteille de NO ou lors de la mise en route du dispositif, lorsqu'une partie du gaz, i.e. de mélange NO/N₂, contenu dans le conduit ou tuyau flexible est évacué via un tube d'évacuation et se retrouve alors dans le local hospitalier ou l'habitable d'un véhicule de transport.

De même, les gaz expirés par le patient contiennent aussi du NO résiduel qui est rejet à l'atmosphère par la valve expiratoire du ventilateur.

Par ailleurs, de façon analogue, du NO peut aussi se retrouver aussi dans l'atmosphère ambiante autour du patient en cas de déconnexion intempestive du ventilateur ou du circuit patient, et/ou en cas de ventilation non invasive (VNI) par masque ou à haut débit nasal via des lunettes respiratoires.

De plus, une partie du NO se retrouvant dans l'atmosphère du local hospitalier ou dans l'habitable d'un véhicule de transport, s'oxyde inévitablement en NO₂ au contact de l'oxygène présent dans l'air.

Comme les composés NO et surtout NO₂ sont toxiques pour le personnel médical ou paramédical, des valeurs maximales d'exposition ont été fixées par les Autorités de Santé. Ainsi, en France, la valeur limite d'exposition professionnelle (VLEP) est fixée à 2 ppm pour le NO et à 0,5 ppm pour le NO₂ pour une exposition de 8h, et à 1 ppm pour une exposition de court terme, par exemple 15 minutes.

Une problématique est que certains soignants, principalement le personnel paramédical, craignent d'être exposés de manière importante au NO et au NO₂ lorsqu'ils réalisent des soins patients traités par administration de NO, ce qui génère de l'anxiété chez ceux-ci et peut par ailleurs affecter leur santé, en particulier si les valeurs maximales d'exposition sont dépassées.

Il existe des équipements de protection individuelle pour monitorer le NO et NO₂ mais ceux-ci sont principalement destinés à être utilisés dans l'industrie car ils ne sont pas ou mal adaptés à une utilisation à l'hôpital et/ou dans l'habitable d'un véhicule de transport, i.e. équipements pas toujours disponibles et/ou fonctionnels, rotation des équipes soignantes, non-connaissance par le personnel soignant de leur existence et/ou de leur lieu de rangement, complexité à les utiliser dans les véhicules d'urgence...

On connait aussi des appareils de NO équipés d'une ligne d'analyse de gaz destinée par exemple à opérer une calibration de l'appareil, par exemple EP4316555, EP4537879, US2017/074845, EP4434565 et EP2522384.

De là, un problème est de pouvoir connaître la concentration de composés potentiellement toxiques, en particulier les composés NO et/ou NO₂, dans l'air ambiant d'un local hospitalier ou de l'habitable d'un véhicule de transport dans lequel se trouve et/ou est utilisée un appareil de délivrance de NO et/ou une (des) installation de fourniture de NO, par exemple une salle de soins, de réanimation, un habitacle d'ambulance, d'hélicoptère, d'avion ou autre, de manière à diminuer l'anxiété des personnels soignants et surtout à préserver leur santé en leur permettant de s'assurer qu'ils ne sont pas soumis à des doses toxiques excessives, typiquement supérieures aux VLEP ou VLEP-CT (court-terme).

Une solution selon l'invention concerne un appareil ou dispositif de délivrance de NO, c'est-à-dire un appareil pour fournir un gaz contenant du NO, typiquement un mélange NO/azote, comprenant :
- un circuit principal de gaz pour véhiculer un gaz contenant du NO, typiquement un mélange NO/N₂, jusqu'à au moins une sortie de gaz configurée pour être raccordée fluidiquement à un circuit patient véhiculant un gaz respiratoire contenant de l'oxygène, tel de l'air ou un mélange oxygène/azote,
- une ligne principale de prélèvement de gaz configurée pour être raccordée fluidiquement audit circuit patient,
- une ligne d'analyse de gaz comprenant des moyens de mesure de concentration configurés pour opérer des mesures de teneur en NO et/ou en NO₂ dans le flux gazeux présent au sein de ladite ligne d'analyse de gaz,
- des moyens d'aspiration de gaz configurés pour aspirer et faire circuler du gaz dans au moins la ligne d'analyse de gaz,
- des moyens de pilotage à microprocesseur configurés pour traiter les mesures de concentration opérées par les moyens de mesure de concentration et en déduire au moins une teneur en NO et/ou en NO₂, et
- des moyens d'affichage configurés pour afficher ladite au moins une teneur en NO et/ou en NO₂.

De plus, selon l'invention, l'appareil comprend en outre une ligne secondaire de prélèvement de gaz communiquant fluidiquement avec l'atmosphère ambiante, i.e. l'air atmosphérique, et des moyens à vanne raccordés fluidiquement à la ligne principale de prélèvement de gaz, à la ligne secondaire de prélèvement de gaz et à la ligne d'analyse de gaz, et les moyens de pilotage sont en outre configurés pour contrôler les moyens à vanne pour mettre la ligne d'analyse de gaz en communication fluidique avec l'une ou, alternativement, l'autre des lignes principale de prélèvement de gaz et secondaire de prélèvement de gaz.

Selon le mode de réalisation considéré, l'appareil de l'invention peut comprendre l'une ou plusieurs des caractéristiques suivantes :
- les moyens à vanne comprennent une électrovanne comprenant : une entrée de vanne principale raccordée fluidiquement à la ligne principale de prélèvement de gaz, une entrée de vanne secondaire raccordée fluidiquement à la ligne secondaire de prélèvement de gaz, et une sortie de vanne raccordée fluidiquement à la ligne d'analyse de gaz.
- les moyens de pilotage sont configurés pour contrôler les moyens à vanne, en particulier l'électrovanne, pour mettre la ligne d'analyse de gaz en communication fluidique (i.e. la commuter) avec :
   ∘ soit la ligne principale de prélèvement de gaz pour alimenter la ligne d'analyse de gaz avec du gaz provenant du circuit patient, c'est-à-dire typiquement un ménage gazeux contenant du NO, de l'azote et de l'oxygène,
   ∘ soit la ligne secondaire de prélèvement de gaz pour alimenter la ligne d'analyse de gaz avec de l'air ambiant provenant de l'atmosphère entourant l'appareil 1, c'est-à-dire de l'air atmosphérique présent dans le local où se trouve l'appareil.
- les moyens à vanne comprennent une électrovanne pilotée.
- l'électrovanne est pilotée, i.e. contrôlée ou commandée, par les moyens de pilotage.
- l'électrovanne est du type à 3 voies, i.e. les 2 entrées de vanne et la sortie de vanne.
- l'électrovanne est reliée électriquement aux moyens de pilotage.
- selon un autre mode de réalisation les moyens à vanne peuvent comprendre plusieurs vannes ou analogues.
- les moyens d'aspiration de gaz comprennent une pompe aspirante comprenant une entrée de gaz reliée fluidiquement à la ligne d'analyse de gaz et une sortie de gaz reliée fluidiquement à l'atmosphère, de préférence via un orifice de sortie ou évent.
- les moyens de pilotage sont configurés pour piloter les moyens d'aspiration de gaz pour aspirer du gaz.
- les moyens d'aspiration de gaz permettent d'aspirer du gaz à un débit d'aspiration donné compris entre 50 et 200 mL/min.
- les moyens de mesure de concentration comprennent un capteur de NO et/ou un capteur de NO₂.
- les moyens d'affichage comprennent un écran d'affichage, de préférence un écran tactile, c'est-à-dire à dalle tactile.
- selon un mode de réalisation, l'écran tactile est configuré pour afficher une touche d'activation actionnable par appui de l'utilisateur, i.e. une touche à actionnement tactile permettant de lancement d'une procédure d'analyse d'air ambiant.
- l'écran tactile est configuré pour afficher une touche de confirmation de choix.
- la touche de confirmation de choix est affichée au sein d'une fenêtre s'affichant sur l'écran, après appui par l'opérateur sur la touche d'activation.
- la touche de confirmation de choix est configurée pour autoriser/démarrer ou interdire/annuler le lancement d'une procédure d'analyse d'air ambiant.
- le lancement de la procédure d'analyse d'air ambiant comprend une action des moyens de pilotage sur les moyens à vanne, en particulier sur l'électrovanne, pour alimenter la ligne d'analyse de gaz par de l'air ambient fourni par la ligne de prélèvement secondaire.
- les moyens de pilotage sont configurés pour agir sur les moyens à vanne, en réponse à un appui par l'utilisateur sur la touche d'activation, de préférence après un autre appui sur la touche de confirmation de choix, pour mettre la ligne d'analyse de gaz en communication fluidique avec la ligne secondaire de prélèvement de gaz de manière à prélever de l'air ambiant et de le soumettre aux moyens de mesure de concentration afin de déterminer la teneur en NO et/ou la teneur en NO₂ de l'air ambiant prélevé.
- selon un autre mode de réalisation, les moyens de pilotage sont configurés pour agir automatiquement (i.e. sans appui ou autre intervention de l'utilisateur) à une fréquence d'activation donnée sur les moyens à vanne pour mettre la ligne d'analyse de gaz en communication fluidique avec la ligne secondaire de prélèvement de gaz de manière à prélever de l'air ambiant et de le soumettre aux moyens de mesure de concentration afin de déterminer la teneur en NO et/ou la teneur en NO₂ de l'air ambiant prélevé, c'est-à-dire pour mesurer de façon périodique la teneur de NO et/ou NO₂ dans la pièce.
- les moyens de pilotage sont configurés pour agir automatiquement sur les moyens à vanne, c'est-à-dire pour activer les moyens à vanne, à une fréquence d'activation de plusieurs fois par jour, de préférence une à plusieurs fois par heure.
- la fréquence d'activation est mémorisée, de préférence dans des moyens de mémorisations et/ou au sein des moyens de pilotage.
- la fréquence d'activation est mémorisée sous forme d'un calendrier.
- l'écran tactile est configuré pour afficher la teneur en NO et/ou la teneur en NO₂ déterminées dans l'air ambiant.
- les moyens de pilotage sont configurés pour déterminer les teneurs en NO et/ou en NO₂ dans l'air ambiant en traitant les mesures opérées par les moyens de mesure de concentration, après un délai ou durée de latence donné.
- le délai ou durée de latence est compris entre de 10 à 120 secondes, de préférence au moins 30 sec, typiquement entre 40 et 90 sec.
- selon un mode de réalisation, le délai ou la durée de latence donné débute après appui par l'utilisateur sur la touche d'activation ou de préférence après un autre appui sur la touche de confirmation de choix.
- selon un autre mode de réalisation, le délai de latence débute après actionnement des moyens à vanne, i.e. actionnement automatique ou actionnement manuel de l'utilisateur par appui sur l'écran tactile.
- selon un mode de réalisation, le délai de latence donné est prédéfini et/ou mémorisé.

Selon le mode de réalisation considéré, l'appareil de l'invention peut aussi comprendre l'une ou plusieurs des caractéristiques additionnelles suivantes :
- les moyens de pilotage sont configurés pour piloter les moyens d'aspiration de gaz, telle une pompe aspirante, pour aspirer du gaz provenant de la ligne principale de prélèvement de gaz ou de la ligne secondaire de prélèvement de gaz.
- la ligne principale de prélèvement de gaz comprend un dispositif anti-retour.
- la ligne secondaire de prélèvement de gaz comprend un moyen de filtration, tel un filtre HEPA ou analogue.
- la ligne secondaire de prélèvement de gaz comprend un dispositif à orifice calibré, de préférence il est agencé entre le moyen de filtration et l'électrovanne.
- la ligne d'analyse de gaz comprend un ou des prises de pression et un dispositif à orifice calibré.
- le débit d'aspiration donné de la pompe aspirante est inférieur ou égal à 400 mL/min, de préférence d'au moins 50 mL/min, de préférence entre 100 et 350 ml/Min, de préférence encore entre 200 et 300 mL/min.
- les moyens d'aspiration de gaz comprennent une pompe aspirante de type diaphragmatique, aussi appelée pompe à diaphragme, ou de type piézoélectrique, c'est-à-dire exploitant lors de son fonctionnement, l'effet piézoélectrique en dynamique résultant de l'excitation fréquentielle.
- les moyens de mesure de concentration comprennent des capteurs de NO et/ou NO₂.
- les moyens de mesure de NO/NO₂ comprennent des capteurs électrochimiques.
- les moyens de mesure de concentration comprennent préférentiellement un capteur d'oxygène, typiquement un capteur d'O₂ électrochimique.
- il comprend des moyens d'alimentation électrique fournissant du courant électrique aux différents éléments ou composants de l'appareil en ayant besoin pour fonctionner, par exemple aux moyens de pilotage, aux capteurs, à la pompe aspirante, etc.... De préférence, les moyens d'alimentation électrique comprennent un raccordement au secteur (110/220V) et/ou une batterie rechargeable ou autre, et éventuellement un convertisseur de courant.
- les moyens de pilotage sont configurées pour traiter les mesures provenant des moyens de mesure de concentration pour en déduire des valeurs de concentration ou teneur en NO et/ou NO₂ dans le flux gazeux analysé.
- les moyens de mesure comprennent en outre un capteur d'O₂ pour mesurer la teneur en O₂ du flux gazeux analysé.
- les moyens d'affichage sont contrôlés par les moyens de pilotage pour afficher les concentrations, i.e. teneurs, en NO et NO₂, et éventuellement en O₂.
- les moyens d'affichage sont contrôlés par les moyens de pilotage pour afficher en permanence les concentrations de NO et de NO₂, et éventuellement en O₂.
- les concentrations de NO et de NO₂ affichées correspondent à celles dans l'air ambiant environnant, i.e. dans la pièce ou autre lieu où se trouve l'appareil.
- les moyens de pilotage sont configurées pour traiter des mesures provenant des moyens de mesure de concentration pour en déduire des valeurs de concentration ou teneur en NO et/ou NO₂ dans le flux gazeux analysé plusieurs fois par jour.
- les moyens de mesure sont configurés pour réaliser une ou plusieurs mesures de concentration ou teneur en NO et/ou NO₂ dans le flux d'air ambiant entre deux calibrations de l'appareil.
- les moyens de mesure sont configurés pour réaliser une ou plusieurs mesures de concentration ou teneur en NO et/ou NO₂ dans le flux d'air ambiant pendant le déroulé d'un traitement de patient.
- les moyens d'affichage sont configurés pour opérer un ou des affichages sur l'écran d'affichage.
- l'écran d'affichage est en couleurs ou en noir et blanc, de préférence en couleurs.
- le circuit principal de gaz de l'appareil de délivrance de NO comprend des moyens de contrôle de débit pour contrôler le débit de gaz dans le circuit principal de gaz véhiculant le mélange NO/N₂, de préférence au moins une électrovanne, notamment de type proportionnel.
- le circuit principal de gaz comprend un ou des passages de gaz, tels des conduits de gaz ou analogues.
- le circuit principal de gaz comprend au moins une entrée de gaz par laquelle le gaz contenant du NO, typiquement un mélange NO/N₂, peut entrer dans le circuit principal de l'appareil.
- la sortie de gaz par laquelle le mélange NO/N₂ sort du circuit principal est reliée au circuit patient par un tuyau flexible ou analogue.
- les moyens de contrôle de débit sont commandés par les moyens de pilotage pour permettre ou empêcher toute circulation de gaz dans le circuit principal de gaz, en particulier ladite au moins une électrovanne est pilotée par les moyens de pilotage (i.e. électrovanne pilotée).
- le circuit principal de gaz comprend en outre des moyens de contrôle de pression, en particulier un dispositif détendeur de gaz.
- les moyens de pilotage comprennent au moins un microprocesseur agencé sur au moins une carte électronique.
- ledit au moins un microprocesseur met en œuvre au moins un algorithme.
- au moins un algorithme est configuré pour déterminer des teneurs en NO et/ou NO₂, voire O₂, à partir de mesures opérées par les moyens de mesure de concentration de la ligne d'analyse de gaz.
- il comprend une coque ou carcasse externe.
- il comprend des moyens de mémorisation, telle une mémoire flash ou analogue.
- les valeurs de teneurs en NO, NO₂ et/ou O₂ peuvent être mémorisées par les moyens de mémorisation.

Par ailleurs, l'invention concerne aussi une installation d'administration de gaz à un patient c'est-à-dire dédiée à la fourniture d'un mélange gazeux contenant du NO et l'oxygène à un patient, i.e. une personne, en ayant besoin, comprenant :
l'appareil de délivrance de NO selon l'invention ; un ventilateur médical, tel un ventilateur de réanimation ; et le circuit patient auquel sont reliés fluidiquement l'appareil de délivrance de NO et le ventilateur médical, et dans laquelle le circuit patient est raccordé fluidiquement aux moyens à vanne, via la ligne principale de prélèvement de gaz.

Selon le mode de réalisation considéré, l'installation de fourniture de gaz de l'invention peut comprendre l'une ou plusieurs des caractéristiques suivantes :
- le ventilateur médical est un appareil d'assistance ventilatoire, tel un ventilateur de réanimation.
- le ventilateur médical alimente le circuit patient en un gaz respiratoire contenant de l'oxygène, typiquement au moins 20% vol. environ d'oxygène, de préférence au moins 20% vol. environ d'oxygène, en particulier de l'air ou un mélange N₂/O₂.
- l'appareil de délivrance de NO est alimenté en un gaz contenant du NO, en particulier en un mélange NO/N₂, par une (ou plusieurs) source de gaz contenant du NO gazeux.
- la (ou les) source de gaz contient un mélange NO/N₂ contenant entre 100 et 2500 ppm en volume de NO (ppmv), le reste étant de l'azote, de préférence moins de 1500 ppmv, le reste étant de l'azote, préférentiellement moins de 1000 ppmv, le reste étant de l'azote.
- préférentiellement, la source de gaz contient un mélange NO/N₂ contenant de 250 à 900 ppm en volume de NO, le reste étant de l'azote, par exemple de l'ordre de 800 ppm en volume de NO, le reste étant de l'azote.
- la (ou les) source de gaz est une (des) bouteille de gaz sous pression.
- le circuit patient comprend une branche inspiratoire et une branche expiratoire.
- l'appareil de délivrance de NO, le ventilateur médical et la ligne principale de prélèvement de gaz de l'appareil de délivrance de NO sont raccordés fluidiquement à la branche inspiratoire du circuit patient.
- la branche inspiratoire et la branche expiratoire sont raccordées au niveau d'une pièce de jonction, telle une pièce en Y.
- le circuit patient, en particulier la branche inspiratoire, alimente une interface respiratoire, telle une sonde d'intubation trachéale ou un masque respiratoire.
- un capteur de débit est agencé sur le circuit patient, de préférence sur la branche inspiratoire.
- le capteur de débit est agencé en amont du site d'injection du NO gazeux, i.e. du mélange NO/N₂, provenant de l'appareil de délivrance de NO.
- un humidificateur de gaz est agencé sur la branche inspiratoire, de préférence en aval du site d'injection du NO gazeux, i.e. du mélange NO/N₂, provenant de l'appareil de délivrance de NO.
- le circuit patient est raccordée fluidiquement à un port de sortie du ventilateur médical de manière à récupérer et acheminer le gaz contenant de l'oxygène délivré par le ventilateur médical, tel de l'air ou un mélange N₂/O₂.
- la (ou les) bouteille de gaz sous pression contient, lorsqu'elle est pleine, un mélange gazeux NO/N₂ à une pression d'au moins 150 à 200 bar abs, voire d'au moins 250 à 300 bar abs.
- le circuit patient comprend des conduites ou tuyaux flexibles.
- le capteur de débit est relié électriquement aux moyens de pilotage pour leur fournir des mesures de débit du flux gazeux provenant du ventilateur médical.
- la ligne principale de prélèvement de gaz de l'appareil de délivrance de NO est raccordée fluidiquement au circuit patient en un site de prélèvement de gaz situé en aval du capteur de débit principal, de préférence en aval de l'humidificateur de gaz.
- le circuit de gaz de l'appareil de délivrance de NO est raccordé fluidiquement au circuit patient via une ligne d'injection de gaz, tel un tuyau flexible.
- la ligne principale de prélèvement de gaz est reliée fluidiquement au circuit patient via un conduit de prélèvement, tel un tuyau flexible.
- le raccordement du conduit de prélèvement au circuit patient se fait au moyen d'une pièce de raccordement, telle un raccord en T, permettant d'opérer des prélèvements de gaz au sein du circuit patient, i.e. dans la branche inspiratoire.
- l'appareil de délivrance de NO alimente le circuit patient en un mélange NO/N₂, de manière à diluer le NO gazeux (i.e. du mélange NO/N₂) dans le flux gaz respiratoire contenant de l'oxygène, tel de l'air ou un mélange O₂/N₂, et obtenir un mélange gazeux final destiné à être administré au patient.
- le mélange gazeux final comprend essentiellement de l'azote (N₂), de l'oxygène (O₂) à une concentration d'au moins 20%vol. environ, et du NO à une teneur comprise entre 1 et 80 ppmv, typiquement de l'ordre de 5 à 40 ppmv correspondant à une posologie souhaitée, i.e. un mélange gazeux NO/O_{2/}N₂.
- le mélange gazeux final peut comprendre des espèces NO₂ résultant de l'oxydation d'une partie du NO, de préférence moins de 2 ppmv de NO₂, de préférence encore moins de 1 ppmv de NO₂.

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les pressions sont exprimées en bar absolus, abréviés « bar ».
- les termes « concentration », « quantité », « proportion », « dose » et « teneur » sont considérés comme équivalents et substituables.
- le terme « local » est à interpréter de manière large. Il peut désigner une pièce, une salle, une chambre, un couloir ou autres dans un établissement hospitalier (i.e. hôpital, clinique ou autre) et aussi l'habitacle ou un compartiment ou espace interne d'un véhicule de transport, tel qu'une ambulance, un hélicoptère, un avion ou autre.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de pression », on entend une valeur de pression (e.g. une valeur numérique) ou un signal représentatif d'une telle valeur de pression reflétant ou correspondant à la pression gazeuse mesurée par un capteur de pression ou analogue.
- par « mesure de teneur en composé A », on entend une valeur de teneur en composé A (e.g. une valeur numérique) ou un signal représentatif d'une telle valeur reflétant ou correspondant à la concentration du composé A dans un flux gazeux analysé.
- les termes « amont » et « aval » sont utilisés par rapport au sens normal de circulation du gaz, à savoir dans le sens allant vers le patient.

L'invention va maintenant être mieux comprise grâce à la description détaillée suivante, faite à titre illustratif mais non limitatif, en référence aux figures annexées parmi lesquelles :
Fig. 1 est une vue schématique d'un mode de réalisation d'une installation d'administration de gaz à un patient comprenant un appareil de délivrance de NO selon l'invention.
Fig. 2 est une représentation schématique des moyens d'analyse de gaz d'un appareil de délivrance de NO selon l'invention, tel celui de l'installation de Fig. 1.
Fig. 3 à Fig. 5 sont des exemples d'affichages opérés par l'appareil de délivrance de NO selon l'invention.

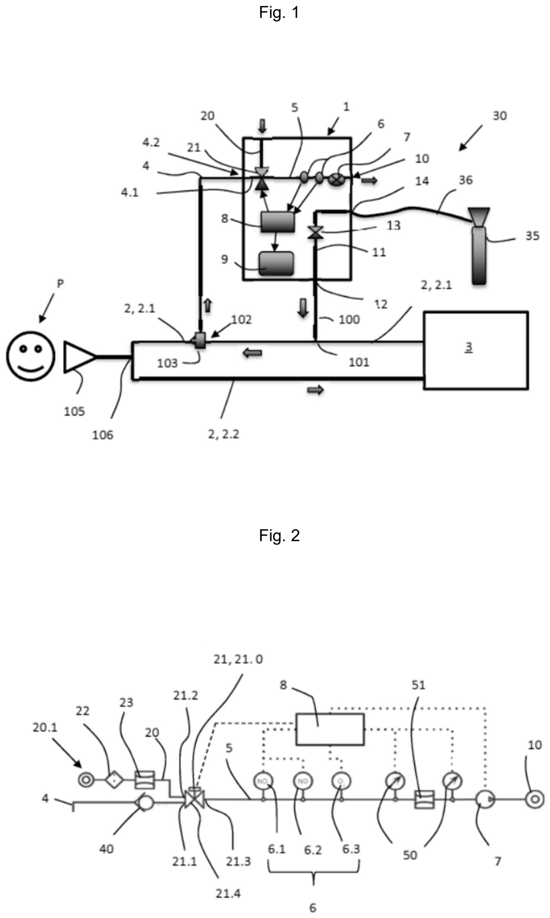

Fig. 1 schématise un mode de réalisation d'une installation de délivrance de gaz 30 permettant de fournir un mélange gazeux contenant du NO à un patient en ayant besoin, comprenant un appareil ou dispositif de délivrance de NO 1 pour fournir un gaz à base de NO, typiquement un mélange gazeux NO/N₂, et un ventilateur médical 3, c'est-à-dire un appareil d'assistance respiratoire délivrant un gaz respiratoire contenant de l'oxygène, typiquement au moins 20 % d'oxygène (O₂) environ, en général d'au moins 21 % d'oxygène environ.

Une telle installation 30 permet de délivrer à un patient P, un mélange gazeux final contenant du NO à une concentration désirée correspondant à une posologie fixée par un médecin-anesthésiste ou analogue, typiquement entre 1 et 80 ppmv de NO (i.e. ppm en volume). Le mélange gazeux final est formé par mélange d'un flux de gaz à base de NO, tel un mélange NO/N₂ provenant de l'appareil de délivrance de NO 1 et d'un flux de gaz respiratoire contenant de l'oxygène, typiquement au moins 20 % d'oxygène (O₂) environ, tel de l'air ou un mélange O₂/N₂, provenant du ventilateur médical 3.

Le flux de gaz à base de NO (i.e. NO/N₂) sortant de l'appareil de délivrance de NO 1 par une sortie 12, est acheminé par un conduit flexible 100 et est ensuite injecté (en 101) dans un circuit patient 2, en particulier dans la branche inspiratoire 2.1 du circuit patient 2, alimenté par ailleurs avec le flux de gaz contenant de l'oxygène fourni par le ventilateur médical 3. Le circuit patient 2 comprend par exemple des tuyaux flexibles, par exemple en polymère.

Le débit du flux de gaz à base d'O₂ circulant dans le circuit patient 2 est mesuré par un capteur de débit principal (non montré), agencé sur le circuit patient 2, i.e. sur la branche inspiratoire 2.1, lequel capteur est relié électriquement aux moyens de pilotage 8 de l'appareil de délivrance de NO 1 afin de leur fournir des signaux ou des mesures de débit reflétant le débit du flux de gaz contenant de l'oxygène fourni par le ventilateur médical 3 et circulant dans le circuit patient 2 en direction du patient P. Le capteur de débit principal peut être un capteur de débit est du type débitmètre massique ou à différentiel de pression, ou autre.

Le mélange gazeux final obtenu par mélange des flux de NO/N₂ et d'air ou de mélange O₂/N₂ est formé essentiellement d'oxygène et d'azote, et de NO à la posologie désirée, typiquement entre 1 et 80 ppmv de NO ; il peut contenir des impuretés inévitables, par exemple de l'argon ou autres. Il est administré au patient P, pendant ses phases inspiratoires, au moyen d'une interface respiratoire 105, par exemple un masque respiratoire, une sonde d'intubation trachéale ou toute autre interface adaptée.

Les gaz expirés par le patient lors de ses phases expiratoires, sont recueillis par une branche expiratoire 2.2 du circuit patient 2. Les branches inspiratoire et expiratoire 2.1, 2.2 comprennent des conduits, canalisations, tuyaux, passages, tubulures ou similaires, par exemple des tuyaux flexibles en polymère, aptes à et configurés pour convoyer les flux gazeux, lesquels sont raccordées fluidiquement à une pièce de jonction 106, telle une pièce en Y ou similaire, laquelle est reliée par ailleurs à l'interface respiratoire 105. L'interface respiratoire 105 permet de délivrer le gaz au patient P, en particulier pendant les phases inspiratoires, et de collecter les gaz expirés (riches en CO₂) par le patient P, en particulier pendant les phases expiratoires.

Pendant le fonctionnement de l'installation 30, le flux de gaz circulant dans la branche inspiratoire 2.1 du circuit patient 2, c'est-à-dire allant du ventilateur mécanique 3 au patient P, est inhalé par le patient, alors que les gaz expirés par celui-ci, i.e. enrichis en CO₂, sont convoyés par la branche expiratoire 2.1 jusqu'au ventilateur 3 où ils sont évacués à l'atmosphère via un orifice de mise à l'atmosphère (non montré) ou analogue.

D'une façon générale, l'appareil de délivrance de NO 1 est d'architecture et de fonctionnement classiques pour ce qui est de la fourniture de NO, typiquement d'un mélange NO/N₂. Il comprend (non montrés) un circuit principal de gaz interne 11 pour véhiculer le gaz contenant du NO, en particulier une (ou des) ligne interne d'acheminement de gaz, typiquement d'un mélange NO/N₂ comprenant de 100 à 2000 ppmv de NO (reste N₂), typiquement moins de 1000 ppmv.

Des moyens de contrôle du débit 13, telle une électrovanne, et/ou de la pression du flux gazeux, tel un dispositif détendeur de gaz, sont agencés sur le circuit principal de gaz 11. Les moyens de contrôle du débit 13 peuvent être pilotés par les moyens de pilotage 8 de l'appareil de délivrance de NO 1, notamment à partir des signaux ou mesures de débit fournis par le capteur de débit principal.

Les moyens de pilotage 8 comprennent un ou des (micro)processeur(s) portés par une (des) carte électronique et mettant en œuvre un ou des algorithmes configurés pour traiter les mesures ou autres informations, données ou analogues.

Le capteur de débit principal est avantageusement agencé sur la branche inspiratoire 2.1, en amont du site d'injection 101 où se fait l'injection de NO (i.e. du mélange NO/azote) dans la branche inspiratoire 2.1 et son mélange avec le flux de gaz contenant de l'oxygène provenant du ventilateur médical 3.

Le mélange NO/N₂ traversant l'appareil de délivrance de NO 1 provient d'une ou plusieurs sources de gaz 35, typiquement une ou des bouteilles de gaz comprimé (non montrées) contenant le mélange gazeux NO/N₂ à une pression pouvant atteindre 150 bar, voire 180 à 200 bar, ou plus. La (ou les) source de gaz 35 vient de raccorder fluidiquement, via un (des) conduit flexible 36, à (au moins) une entrée de gaz 14 du circuit principal de gaz 11 du dispositif de délivrance de NO 1.

Comme déjà expliqué, l'appareil de délivrance de NO 1 permet de fournir/d'injecter (en 101) le mélange à base de NO (i.e. NO/azote) dans le circuit patient 2 afin qu'il s'y mélange (i.e. dilution) avec le flux de gaz contenant de l'oxygène provenant du ventilateur médical 2, par exemple un mélange N₂/O₂ ou de l'air.

Le mélange gazeux final NO/O₂/N₂ qui est ensuite administré au patient P via l'interface respiratoire 105, comprend principalement de l'azote (N₂), de l'oxygène (O₂) à une teneur d'au moins 20%vol. environ, et du NO à une teneur comprise entre 1 et 80 ppmv, typiquement de l'ordre de 5 à 40 ppmv correspondant à la posologie désirée, laquelle dépend notamment de la population traitée, i.e. nouveau-nés, enfants, adolescents ou adultes, et de la maladie à traiter.

En effet, le monoxyde d'azote ou NO, lorsqu'il est inhalé, dilate les vaisseaux pulmonaires et augmente l'oxygénation en améliorant les échanges gazeux. Ces propriétés sont utilisées pour traiter différentes conditions médicales, comme l'Hypertension Artérielle Pulmonaire du Nouveau-né ou PPHN (pour *Persistent Pulmonary Hypertension of the Newborn*), le Syndrome de Détresse Respiratoire Aigüe ou SDRA observé principalement chez l'adulte ou encore les hypertensions pulmonaires (HP) en chirurgie cardiaque chez l'adulte ou l'enfant, comme décrit notamment par EP-A-560928, EP-A-1516639 et US-A-10,201,564.

Toutefois, du fait de la présence d'O₂ dans le mélange gazeux final NO/O₂/N₂, une partie du NO qui s'y trouve peut s'oxyder et former des espèces NO₂ toxiques.

Il est dès lors primordial de s'assurer que la concentration en NO dans le mélange gazeux final NO/O₂/N₂ correspond bien à la posologie souhaitée par le médecin et que, par ailleurs, la quantité d'espèces NO₂ toxiques soit limitée, typiquement inférieure à quelques ppmv, en général moins de 0.5 ppmv.

Pour ce faire, l'appareil de délivrance de NO 1 de Fig.1 intègre des moyens d'analyse de gaz 5, 6, i.e. un analyseur de gaz, qui sont agencé dans la coque ou carcasse externe, par exemple en polymère, de l'appareil de délivrance de NO 1, et qui permettant de monitorer, i.e. mesurer, les concentrations en NO et NO₂ dans le mélange gazeux final, comme expliqué ci-après.

Comme détaillé en Fig. 2, les moyens d'analyse de gaz 5, 6 comprennent une ligne d'analyse de gaz 5 comprenant des moyens de mesure de concentration 6 configurés pour opérer des mesures de teneur en NO et/ou en NO₂, voire aussi en oxygène, dans le flux gazeux présent au sein de ladite ligne d'analyse de gaz 5.

Une ligne principale de prélèvement de gaz 4, tel un tuyau flexible, raccorde fluidiquement le circuit patient 2 à la ligne d'analyse de gaz 5 de manière à permettre de prélever des échantillons gazeux dans le circuit 2, i.e. dans la branche inspiratoire 2.1, et de les acheminer jusqu'à la ligne d'analyse de gaz 5 afin de les mettre en contact avec les moyens de mesure de concentration 6 afin qu'ils puissent opérer des mesures de teneurs en NO et NO₂ et/ou O₂.

De préférence, les moyens de mesure de concentration 6 comprennent des capteurs de NO, de NO₂ et éventuellement d'O₂, par exemple de type électrochimique ou autre. Les capteurs peuvent être dédiés chacun à un composé donné (NO, NO₂ ou O₂) ou être combinés pour mesurer les teneurs en plusieurs composés, en particulier NO et NO₂.

Les mesures de concentration opérées par les moyens de mesure de concentration 6 sont transmissent aux moyens de pilotage 8 qui les traitent afin d'en déduire des valeurs de teneurs en NO et NO₂ et/ou O₂.

Afin de permettre un prélèvement de gaz, c'est-à-dire des échantillons gazeux, dans le circuit 2, des moyens d'aspiration de gaz 7, telle une pompe aspirante, sont agencés en aval de la ligne principale de prélèvement de gaz 4, typiquement dans la ligne d'analyse de gaz 5, de manière d'aspirer ces prélèvements de gaz. La pompe aspirante peut être de type à diaphragme ou piézoélectrique.

Comme illustré en Fig. 2, la ligne d'analyse de gaz 5 peut aussi comprendre des prises ou capteurs de pression 50 agencées de part et d'autre d'un dispositif à orifice calibré 51, notamment pour constituer un capteur de pression différentiel, lesquelles sont reliées électriquement aux moyens de pilotage 8 qui peuvent être configurés pour déterminer un débit gazeux à partir des mesures de pression différentielles.

Par ailleurs, l'appareil de délivrance de NO 1 comprend aussi des moyens d'affichage permettant d'afficher différentes informations, valeurs, données, alarmes, graphiques, icones, fenêtres, courbes, jauges ou autres. De préférence, ils comprennent un écran d'affichage 9, de préférence un écran tactile, c'est-à-dire à dalle tactile. Les affichages se font préférentiellement en couleurs.

En particulier, l'écran tactile 9 permet aussi d'afficher une ou des touches virtuelles à actionnement tactile afin d'opérer notamment une ou des activations, sélections ou validations, par exemple dans des menus de sélection ou dans des fenêtres virtuelles s'affichant également sur l'écran 9. Une (des) telle touche virtuelle est actionnable par l'utilisateur par appui sur la vitre/dalle de l'écran 9, i.e. activable ou sélectionnable par contact digital ou pression du doigt par l'utilisateur, i.e. le personnel soignant.

Ainsi, l'écran tactile 9 permet aussi d'afficher les teneurs en NO et/ou en NO₂, et éventuellement en O₂, déterminées par les moyens de pilotage 8 à partir des mesures opérées par les moyens de mesure de concentration 6, typiquement par les capteurs de NO, de NO₂ et éventuellement d'O₂. Cet affichage permet au personnel soignant de s'assurer que le mélange gazeux final e.g. (NO/N₂/O₂) contient la posologie désirée en NO, une teneur en oxygène non-hypoxique (i.e. >20%vol.) et à l'inverse, ne contient pas une quantité excessive d'espèces toxiques NO₂.

Les moyens de pilotage 8, en particulier le (les) microprocesseur, sont aussi configurés pour contrôler tout ou partie des affichages qui s'opèrent sur l'écran d'affichage 9, i.e. sur les moyens d'affichage, en particulier les valeurs de teneurs en NO et en NO₂ ayant été déterminées.

L'alimentation électrique de l'appareil de délivrance de NO 1, en particulier des moyens de pilotage 8, les capteurs, la pompe 7, l'écran 9..., est assurée par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant.

L'appareil 1 peut aussi comprendre des moyens de mémorisation, telle une mémoire flash ou analogue. Elle peut être intégrée aux moyens de pilotage 8, par exemple portée par une carte électronique.

Dans le mode de réalisation de Fig. 1, la ligne principale de prélèvement de gaz 4, tel un tuyau flexible, vient se raccorder, via son extrémité aval, à la ligne d'analyse de gaz 5 par l'intermédiaire d'un tronçon de liaison 4.1 situé dans l'appareil 1. Le tronçon de liaison 4.1 comprend un orifice d'entrée 4.2 au niveau duquel vient se raccorder la ligne principale de prélèvement de gaz 4, par exemple l'orifice d'entrée 4.2 peut être porté par un raccord ou un connecteur fluidique ou analogue. Selon un autre mode de réalisation (non montré), la ligne principale de prélèvement de gaz 4 peut se raccorder directement à la ligne d'analyse de gaz 5.

Le raccordement de l'extrémité amont de la ligne principale de prélèvement de gaz 4 au circuit patient 2 se fait (en 102) via un dispositif de raccordement adapté 103, par exemple un connecteur fluidique en T ou analogue agencé en aval du point d'injection 101 de NO dans le circuit patient.

Les moyens d'aspiration de gaz 7, typiquement une pompe aspirante, permet de faire circuler le gaz dans la ligne principale de prélèvement de gaz 4 et dans la ligne d'analyse de gaz 5, via le tronçon 4.1, en créant une dépression engendrant un débit ou flux gazeux qui va venir balayer les moyens de mesure de concentration 6 en NO et en NO₂ (voire aussi en O₂), c'est-à-dire les capteurs de la ligne d'analyse de gaz 5, de manière à ce que ceux-ci puissent opérer les mesures de concentration désirées. Le débit d'aspiration est préférentiellement compris entre 50 et 350 mL/min, par exemple de l'ordre de 200 à 250 mL/min.

Le flux de gaz à analyser est donc aspiré par la pompe 7 et circule dans la ligne d'analyse de gaz 5, pour être mis en contact avec les capteurs de NO₂ et de NO, lesquels vont alors opérer une mesure de la concentration en NO₂ et en NO du flux gazeux et fournir ces mesures aux moyens de pilotage 8 qui vont alors les restituer à l'utilisateur, tel un médecin ou un autre personnel soignant, en commandant leur affichage sur l'écran tactile 9, lequel peut faire partie d'interface graphique utilisateur (IGU) de l'appareil de délivrance de NO 1.

La ligne d'analyse de gaz 110 peut aussi comprendre un ou plusieurs autres capteurs, en particulier un capteur d'oxygène et la concentration d'oxygène peut aussi être affichée sur l'écran 9 afin de permettre à l'utilisateur de la suivre et s'assurer que le mélange gazeux fourni au patient n'est pas hypoxique.

Bien entendu, si besoin, les moyens de pilotage 8 peuvent aussi traiter les mesures de NO et NO₂ avant de commander leur affichage sur l'écran 9, en particulier réaliser une compensation de ces valeurs pour tenir compte de facteurs environnementaux pouvant influencer les mesures, telle la pression atmosphérique, la température et/ou l'humidité, auxquels le mélange gazeux a pu être soumis.

Avantageusement, les moyens de pilotage 8 comprennent une (des) carte de commande électronique et une unité de contrôle à (micro)processeur(s), typiquement un microcontrôleur ou analogue, de préférence la carte de commande électronique intègre l'unité de contrôle. De préférence, les moyens de pilotage 8 commandent, i.e. pilotent, aussi la pompe aspirante 7 afin que le débit circulant dans la ligne d'analyse de gaz 5 soit maintenu constant et égal au débit cible souhaité, e.g. compris entre 50 et 350 mL/min.

Après analyse, le flux de gaz est rejeté à l'atmosphère après avoir transité dans la ligne d'analyse de gaz 5 et au travers de la pompe aspirante 7 située entre les moyens de mesure de concentration 6, i.e. les capteurs NO et NO₂ (voire aussi de O₂), et un orifice de sortie ou évent 10 en communication fluidique avec l'air ambient, i.e. l'atmosphère de la pièce où se trouve l'appareil 1.

L'air ambiant se retrouve donc 'pollué' avec ces composés NO et NO₂.

Or, certains personnels soignants, principalement le personnel paramédical, craignent d'être exposés au NO et au NO₂ lorsqu'ils réalisent des soins dans la pièce où le ou les patients sont traités par administration de NO, par exemple dans une salle de soins ou de réanimation, ce qui génère chez ceux-ci de l'anxiété et peut affecter leur santé.

Dès lors, afin de tenter d'y remédier, selon l'invention, l'appareil de délivrance de NO a été améliorer de manière à pouvoir déterminer et afficher la concentration des composés NO et/ou NO₂ dans l'air ambiant du local hospitalier où se trouve et/ou est utilisée l'installation de fourniture de NO 30, par exemple une salle de soins, de réanimation ou autre.

En effet, connaître leur niveau d'exposition aux composés NO et NO₂ présents dans l'air ambiant, permet de diminuer l'anxiété des personnels soignants et par ailleurs de préserver leur santé en leur permettant de s'assurer qu'ils ne sont pas soumis à des doses excessives, typiquement supérieures aux VLEP.

Pour ce faire, selon l'invention, comme visible en Fig. 1 et détaillé en Fig. 2, ont été prévus dans l'appareil de délivrance de NO 1, une ligne secondaire de prélèvement de gaz 20 communiquant fluidiquement avec l'atmosphère ambiante, au travers d'une entrée d'air ambiant 20.1, et des moyens à vanne 21 raccordés fluidiquement à la ligne principale de prélèvement de gaz 4, à la ligne secondaire de prélèvement de gaz 20, et à la ligne d'analyse de gaz 5.

La ligne principale de prélèvement de gaz 4 comprend ici un dispositif anti-retour 40 agencé en amont des moyens à vanne 21 afin d'empêcher toute remonte de flux gazeux vers le circuit patient 2.

Les moyens à vanne 21 comprennent une électrovanne commandée, i.e. pilotée, par les moyens de pilotage 8, par exemple une électrovanne du type à 3 voies. Elle comprend typiquement un corps de vanne 21.4 avec des passages de gaz internes (non visibles) comprenant : une entrée de vanne principale 21.1 raccordée fluidiquement à la ligne principale de prélèvement de gaz 4 ; une entrée de vanne secondaire 21.2 raccordée fluidiquement à la ligne secondaire de prélèvement de gaz 20 ; et une sortie de vanne 21.3 raccordée fluidiquement à la ligne d'analyse de gaz 5.

Par ailleurs, comme illustré sur Fig. 2, on voit que la ligne secondaire de prélèvement de gaz 20 comprend préférentiellement :
- un moyen de filtration 22, tel un filtre HEPA ou analogue, situé en aval de l'entrée d'air 20.1, servant à purifier l'air en éliminant les poussières, spores, bactéries ou autres aérosols susceptibles de s'y trouver ; et
- un dispositif à orifice calibré 23, agencé entre le moyen de filtration 22 et l'électrovanne 21.0, lequel permet de générer (lorsque cela est nécessaire) une perte de charge du flux d'air dans la ligne secondaire 20 équivalente à celle se produisant dans la ligne principale 4, par exemple lorsque celle-ci est équipée par exemple d'un dispositif de type « piège à eau » (non montré). En effet, un tel « équilibrage » permet à la pompe 7 de rester au même régime lors de la commutation des moyens à vanne 21, typiquement de l'électrovanne 21.0.

Plus précisément, les moyens de pilotage 8 sont configurés pour contrôler aussi les moyens à vanne 21, i.e. l'électrovanne 21.0, pour mettre la ligne d'analyse de gaz 5 en communication fluidique avec l'une ou, alternativement, l'autre des lignes principale et secondaire de prélèvement de gaz 4, 20, c'est-à-dire l'une ou l'autre mais jamais les deux simultanément.

Pour ce faire, les moyens de pilotage 8 sont reliés électriquement auxdits moyens à vanne 21 afin de leur transmettre des signaux de commande visant à opérer un basculement de l'alimentation en gaz de l'électrovanne 21.0 vers l'une ou l'autre des lignes principale et secondaire de prélèvement de gaz 4, 20 selon que l'on souhaite connaître la composition en NO et/ou NO₂ du flux gazeux final provenant du circuit patient 2 ou de l'air ambiant entourant l'appareil 1.

Plus précisément, les moyens à vanne 21, à savoir l'électrovanne 21.0, sont commandés par les moyens de pilotage 8 pour relier fluidiquement la ligne d'analyse de gaz 5 à la ligne principale de prélèvement de gaz 4. A ce moment, le gaz provenant du circuit patient 2 peut entrer dans le corps de l'électrovanne 21.4 par l'entrée de vanne principale 21.1 puis traverser le corps de l'électrovanne 21.4 via ses passages internes, avant d'en ressortir par la sortie de vanne 21.3 raccordée fluidiquement à la ligne d'analyse de gaz 5, de sorte d'être analysé par les moyens de mesure de concentration 6 afin de mesurer la teneur en NO et NO₂ comme expliqué ci-avant, voire celle en O₂, c'est-à-dire grâce aux capteurs de NO 6.1, de NO₂ 6.2 et d'O₂ 6.3 de la ligne d'analyse de gaz 5.

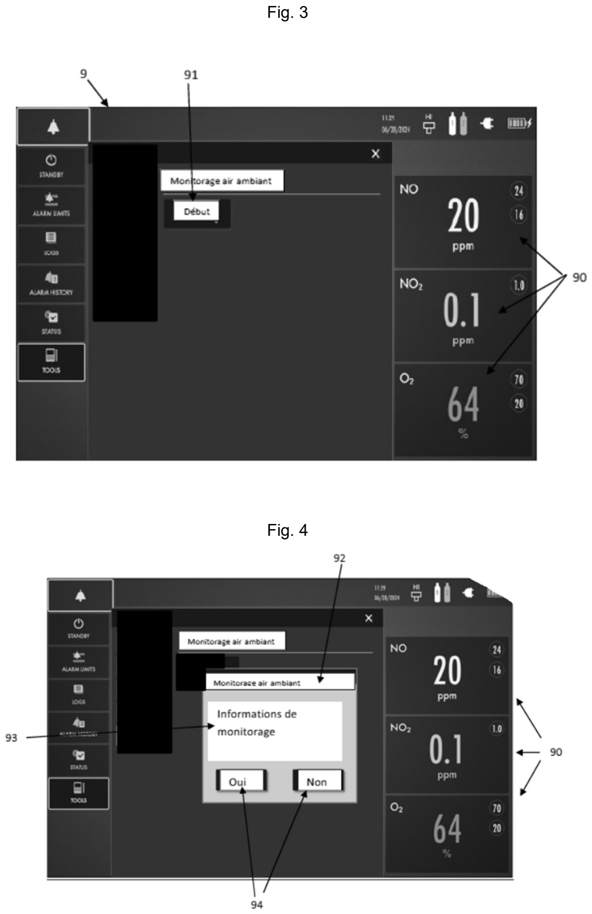

Une fois les mesures opérées par les capteurs de NO 6.1, de NO₂ 6.2 et d'O₂ 6.3 transmises aux moyens de pilotage 8, elles sont traitées par ces derniers qui contrôlent par ailleurs leur affichage (en 90) sur l'écran 9, pendant le déroulé du traitement du patient P, comme illustré en Fig. 3 et Fig. 4, ce qui permet au personnel soignant de l'assurer que la composition du gaz finale fournie au patient est bien conforme à la posologie attendue et ne contient pas d'espèces NO₂ toxiques au-delà du ou des seuils maximum admissibles.

Ceci correspond à un fonctionnement normal et classique de l'appareil de délivrance de NO 1.

Lorsqu'un personnel soignant souhaite connaître son niveau d'exposition aux composés NO et/ou NO₂ présents dans l'air ambiant, c'est-à-dire dans le local hospitalier, un habitacle ou autre où se trouve l'installation 30 et l'appareil 1, afin de s'assurer qu'il n'est pas soumis à des doses excessives de ces composés, selon un mode de réalisation de l'invention, il peut opérer un appui sur l'écran tactile 9 de l'appareil de délivrance de NO 1, en particulier sur une touche d'activation 91 dédiée, par exemple une touche intitulée « Début », comme illustré en Fig. 3.

Optionnellement, comme illustré en Fig. 4, l'appui sur la touche d'activation 91 peut ouvrir une fenêtre 92 sur l'écran d'affichage 9 au sein de laquelle s'affichent des informations (en 93) relatives au monitorage de l'air ambiant et deux touches de confirmation de choix 94, i.e. touche 'oui' et touche 'non' ou similaire, permettant de valider le lancement de la procédure d'analyse de l'air ambiant ou son annulation.

Dans tous les cas, le (ou les) appui du doigt, i.e. pression ou contact digital, sur la dalle tactile de l'écran 9, c'est-à-dire sur la touche d'activation 91 et optionnellement sur l'une ou l'autre des touches de confirmation de choix 94, va (vont) alors être détecté par les moyens de pilotage 8, par exemple via un signal d'activation transmis par la dalle tactile de l'écran 9 auxdits moyens de pilotage 8, lesquels vont alors agir sur les moyens à vanne 21, en réponse à un appui de l'utilisateur sur la touche d'activation 91 (et éventuellement ensuite sur celle de validation de choix 94, i.e. « oui »), pour mettre la ligne d'analyse de gaz 5 en communication fluidique avec la ligne secondaire de prélèvement de gaz 20 de manière à prélever permettre un prélèvement d'air ambiant et de le soumettre, de préférence après filtration (en 22), aux moyens de mesure de concentration 6, i.e. en particulier aux capteurs de NO 6.1 et de NO₂ 6.2, afin de permettre une mesure ou détermination la concentration en NO et en NO₂ de l'air ambiant prélevé.

Selon un autre mode de réalisation, les moyens de pilotage 8 peuvent être configurés pour agir automatiquement à une fréquence d'activation donnée sur les moyens à vanne 21 pour mettre la ligne d'analyse de gaz 5 en communication fluidique avec la ligne secondaire 20 de prélèvement de gaz pour prélever de l'air ambiant et le soumettre aux moyens de mesure de concentration afin de déterminer la teneur en NO et/ou la teneur en NO₂ de l'air ambiant prélevé, comme déjà dit.

Dans ce mode de réalisation, l'appareil de délivrance de 1 opère donc des mesures périodiques des teneurs de NO et/ou NO₂ dans la pièce en agissant automatiquement sur les moyens à vanne 21 pour les activer à une fréquence d'activation donnée, typiquement de plusieurs fois par jour, de préférence une à plusieurs fois par heure.

La fréquence d'activation est mémorisée, par exemple sous forme d'un calendrier journalier ou analogue, de préférence dans des moyens de mémorisations et/ou directement au sein des moyens de pilotage 8, par exemple dans un algorithme dédié.

Autrement dit, en réponse à l'appui sur la touche d'activation 91 (et éventuellement sur celle de validation) ou en fonction de la fréquence d'activation définie, l'électrovanne 21.0 commute afin de stopper le passage du gaz final provenant du circuit patient 2 au travers du corps de l'électrovanne 21.4 par l'entrée de vanne principale 21.1 et, à l'inverse, d'autoriser le passage d'air ambiant amené par la ligne secondaire de prélèvement de gaz 20 au travers du corps de l'électrovanne 21.4 par l'entrée de vanne secondaire 21.2. Le flux d'air ambiant, qui est aspiré par les moyens d'aspiration 7, comme déjà expliqué, est d'abord filtré par le moyen de filtration 22 et peut ensuite traverser le corps de l'électrovanne 21.4, via des passages internes, avant d'en ressortir par la sortie de vanne 21.3 raccordée fluidiquement à la ligne d'analyse de gaz 5 et permettre alors une analyse de la composition de l'air.

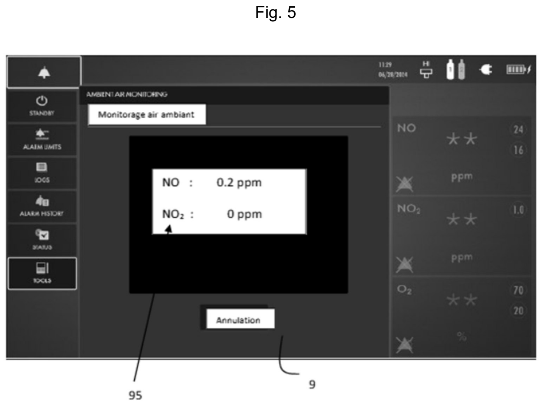

Comme précédemment, une fois les mesures opérées par les capteurs de NO 6.1 et de NO₂ 6.2, elles sont transmises aux moyens de pilotage 8 qui les traitent et contrôlent par ailleurs leur affichage (en 95) sur l'écran 9, comme illustré en Fig. 5, de manière à permettre au personnel soignant de connaître la composition de l'air ambiant en composés NO et NO₂.

Dans ce cas, les valeurs de concentration en NO, NO₂ et O₂ qui avaient été précédemment mesurées dans le flux de gaz final provenant du circuit patient ne sont plus affichées (en 90), contrairement aux Fig. 3 et Fig. 4.

D'une façon générale, les valeurs de NO et NO₂ mesurées dans l'air ambiant peuvent être affichées (en 95) en quasi-temps réel, en réponse à un choix/appui de l'utilisateur ou, selon le mode de réalisation, automatiquement à la fréquence d'activation mémorisée.

Toutefois, l'affichage 95 se fait préférentiellement en léger différé, c'est-à-dire après une durée de latence allant de 10 à 60 secondes, de préférence au moins 30 sec, par exemple de l'ordre de 40 à 45 sec, après activation des moyens à vanne 21 par les moyens de pilotage 8, pour permettre aux capteurs de NO et NO₂ 6.1, 6.2 de « stabiliser » les valeurs mesurées, c'est-à-dire de limiter les fluctuations de mesure résultant d'une présence résiduelle de gaz final provenant du circuit patient 2 susceptible de se mélanger au flux d'air atmosphérique filtré entrant dans la ligne d'analyse 5.

Or, le mélange entre ces deux flux en début de mesure, du fait de la dilution inévitable d'une partie du gaz final (i.e. NO/O₂/N₂) dans la ligne d'analyse 5 par le flux d'air filtré entrant dans la ligne d'analyse, après commutation de l'électrovanne 21.0 des moyens à vanne 21, peut engendrer de légères fluctuations de composition du gaz, donc fausser légèrement les mesures.

Il est donc recommandé d'attendre que la ligne d'analyse 5 ne contiennent plus que de l'air ambiant filtré, c'est-à-dire que le gaz final ait été évacué à l'atmosphère via la pompe 7 et l'orifice de sortie ou évent 10.

En général, on considère qu'après 1 à 2 minutes, les mesures opérées par les capteurs de NO et NO₂ 6.1, 6.2 peuvent être considérées comme 'stabilisées' et reflétant bien les teneurs en NO et NO₂ présents dans l'air ambiant de la pièce. Ces valeurs peuvent être aussi enregistrées par les moyens de mémorisation ou tout autre moyen de sauvegarde.

D'une façon générale, l'installation d'administration de gaz 30 comprenant l'appareil de délivrance de NO 1 selon l'invention est adaptée à une utilisation pour traiter différentes pathologies pulmonaires, comme l'Hypertension Artérielle Pulmonaire du Nouveau-né (PPHN), le Syndrome de Détresse Respiratoire Aigüe (SDRA) et/ou les hypertensions pulmonaires (HP) en chirurgie cardiaque et ce, chez différentes populations de patient, en particulier les nouveau-nés, les adultes, les adolescents ou les enfants.

## Revendications

1. Appareil de délivrance de NO (1) comprenant :
- un circuit principal de gaz (11) pour véhiculer un gaz contenant du NO jusqu'à au moins une sortie de gaz (12) configurée pour être raccordée fluidiquement à un circuit patient (2) véhiculant un gaz respiratoire contenant de l'oxygène,
- une ligne principale de prélèvement de gaz (4) configurée pour être raccordée fluidiquement audit circuit patient (2),
- une ligne d'analyse de gaz (5) comprenant des moyens de mesure de concentration (6) configurés pour opérer des mesures de teneur en NO et/ou en NO₂ dans le flux gazeux présent au sein de ladite ligne d'analyse de gaz (5),
- des moyens d'aspiration de gaz (7) configurés pour aspirer et faire circuler du gaz dans au moins la ligne d'analyse de gaz (5),
- des moyens de pilotage (8) à microprocesseur configurés pour traiter les mesures de concentration opérées par les moyens de mesure de concentration (6) et en déduire au moins une teneur en NO et/ou en NO₂, et
- des moyens d'affichage (9) configurés pour afficher ladite au moins une teneur en NO et/ou en NO₂,
**caractérisé en ce qu'**il comprend en outre :
- une ligne secondaire de prélèvement de gaz (20) communiquant fluidiquement avec l'atmosphère ambiante, et
- des moyens à vanne (21) raccordés fluidiquement à la ligne principale de prélèvement de gaz (4), à la ligne secondaire de prélèvement de gaz (20), et à la ligne d'analyse de gaz (5), les moyens de pilotage (8) étant en outre configurés pour contrôler les moyens à vanne (21) pour mettre la ligne d'analyse de gaz (5) en communication fluidique avec l'une ou, alternativement, l'autre des lignes principale de prélèvement de gaz (4) et secondaire de prélèvement de gaz (20).

2. Appareil selon la revendication 1, **caractérisé en ce que** les moyens à vanne (21) comprennent une électrovanne (21.0) comprenant :
- une entrée de vanne principale (21.1) raccordée fluidiquement à la ligne principale de prélèvement de gaz (4),
- une entrée de vanne secondaire (21.2) raccordée fluidiquement à la ligne secondaire de prélèvement de gaz (20), et
- une sortie de vanne (21.3) raccordée fluidiquement à la ligne d'analyse de gaz (5).

3. Appareil selon la revendication 1, **caractérisé en ce que** les moyens de pilotage (8) sont configurés pour contrôler les moyens à vanne (21), en particulier l'électrovanne (21.0), pour mettre la ligne d'analyse de gaz (5) en communication fluidique avec :
- soit la ligne principale de prélèvement de gaz (4) pour alimenter la ligne d'analyse de gaz (5) avec du gaz provenant du circuit patient (2),
- soit la ligne secondaire de prélèvement de gaz (20) pour alimenter la ligne d'analyse de gaz (5) avec de l'air ambiant provenant de l'atmosphère , i.e. l'air dans le local et entourant l'appareil.

4. Appareil selon la revendication 1, **caractérisé en ce que** les moyens d'aspiration de gaz (9) comprennent une pompe aspirante comprenant une entrée de gaz reliée fluidiquement à la ligne d'analyse de gaz (5) et une sortie de gaz reliée fluidiquement à l'atmosphère, de préférence via un orifice de sortie (10).

5. Appareil selon l'une des revendications 1 ou 4, **caractérisé en ce que** les moyens de pilotage (8) sont configurés pour piloter les moyens d'aspiration de gaz (7) pour aspirer du gaz, de préférence à un débit d'aspiration donné compris entre 50 et 350 mL/min.

6. Appareil selon la revendication 1, **caractérisé en ce que** les moyens de mesure de concentration (6) comprennent un capteur de NO (6.1) et/ou un capteur de NO₂ (6.2).

7. Appareil selon la revendication 1, **caractérisé en ce que** les moyens d'affichage comprennent un écran d'affichage (9) tactile.

8. Appareil selon l'une des revendications 1 et 7, **caractérisé en ce que** :
- l'écran tactile (9) est configuré pour afficher une touche d'activation (91), et éventuellement une touche de confirmation de choix (94), actionnable par appui de l'utilisateur et
- les moyens de pilotage (8) sont configurés pour agir sur les moyens à vanne (21), en réponse à un appui par l'utilisateur sur la touche d'activation (91), et éventuellement sur la touche de confirmation de choix (94), pour mettre la ligne d'analyse de gaz (5) en communication fluidique avec la ligne secondaire de prélèvement de gaz (20) de manière à prélever de l'air ambiant et de le soumettre aux moyens de mesure de concentration (6) afin de déterminer la teneur en NO et/ou la teneur en NO₂ de l'air ambiant prélevé.

9. Appareil selon la revendication 1, **caractérisé en ce que** les moyens de pilotage (8) sont configurés pour agir automatiquement à une fréquence d'activation donnée sur les moyens à vanne (21) pour mettre la ligne d'analyse de gaz (5) en communication fluidique avec la ligne secondaire de prélèvement de gaz (20) de manière à prélever de l'air ambiant et de le soumettre aux moyens de mesure de concentration (6) afin de déterminer la teneur en NO et/ou la teneur en NO₂ de l'air ambiant prélevé.

10. Appareil selon la revendication 9, **caractérisé en ce que** la fréquence d'activation mémorisée est de plusieurs fois par jour, de préférence une à plusieurs fois par heure.

11. Appareil selon l'une des revendications 1 et 7 ou 8, **caractérisé en ce que** l'écran tactile (9) est configuré pour afficher (95) la teneur en NO et/ou la teneur en NO₂ déterminées dans l'air ambiant.

12. Appareil selon l'une des revendications 1 et 8 ou 9, **caractérisé en ce que** les moyens de pilotage (8) sont configurés pour déterminer les teneurs en NO et/ou en NO₂ dans l'air ambiant en traitant les mesures opérées par les moyens de mesure de concentration (6), après un délai de latence donné.

13. Appareil selon la revendication 12, **caractérisé en ce que** le délai de latence est compris entre de 10 à 120 secondes.

14. Appareil selon la revendication 10, **caractérisé en ce que** la fréquence d'activation est mémorisée sous forme d'un calendrier journalier.

15. Installation d'administration de gaz (100) à un patient (P) comprenant :
l'appareil de délivrance de NO (1) selon l'une des revendications 1 à 14 ; un ventilateur médical (3) ; et le circuit patient (2) auquel sont reliés fluidiquement l'appareil de délivrance de NO (1) et le ventilateur médical (3), et dans laquelle le circuit patient (2) est raccordé fluidiquement aux moyens à vanne (20), via la ligne principale de prélèvement de gaz (4).

## Revendications modifiées

### Revendications modifiées conformément à la règle 137(2) CBE.

1. Appareil de délivrance de NO (1) comprenant :
- un circuit principal de gaz (11) pour véhiculer un gaz contenant du NO jusqu'à au moins une sortie de gaz (12) configurée pour être raccordée fluidiquement à un circuit patient (2) véhiculant un gaz respiratoire contenant de l'oxygène,
- une ligne principale de prélèvement de gaz (4) configurée pour être raccordée fluidiquement audit circuit patient (2),
- une ligne d'analyse de gaz (5) comprenant des moyens de mesure de concentration (6) configurés pour opérer des mesures de teneur en NO et/ou en NO₂ dans le flux gazeux présent au sein de ladite ligne d'analyse de gaz (5),
- des moyens d'aspiration de gaz (7) configurés pour aspirer et faire circuler du gaz dans au moins la ligne d'analyse de gaz (5),
- des moyens de pilotage (8) à microprocesseur configurés pour traiter les mesures de concentration opérées par les moyens de mesure de concentration (6) et en déduire au moins une teneur en NO et/ou en NO₂, et
- des moyens d'affichage (9) configurés pour afficher ladite au moins une teneur en NO et/ou en NO₂, lesdits moyens d'affichage (9) comprenant un écran d'affichage (9) tactile,
- une ligne secondaire de prélèvement de gaz (20) communiquant fluidiquement avec l'atmosphère ambiante, et
- des moyens à vanne (21) raccordés fluidiquement à la ligne principale de prélèvement de gaz (4), à la ligne secondaire de prélèvement de gaz (20), et à la ligne d'analyse de gaz (5), les moyens de pilotage (8) étant en outre configurés pour contrôler les moyens à vanne (21) pour mettre la ligne d'analyse de gaz (5) en communication fluidique avec l'une ou, alternativement, l'autre des lignes principale de prélèvement de gaz (4) et secondaire de prélèvement de gaz (20),
**caractérisé en ce que** :
- l'écran tactile (9) est configuré pour afficher une touche d'activation (91), actionnable par appui d'un utilisateur et
- les moyens de pilotage (8) sont configurés pour agir sur les moyens à vanne (21), en réponse à un appui par l'utilisateur sur la touche d'activation (91) pour mettre la ligne d'analyse de gaz (5) en communication fluidique avec la ligne secondaire de prélèvement de gaz (20) de manière à prélever de l'air ambiant et de le soumettre aux moyens de mesure de concentration (6) afin de déterminer la teneur en NO et/ou la teneur en NO₂ de l'air ambiant prélevé.

2. Appareil selon la revendication 1, **caractérisé en ce que** les moyens à vanne (21) comprennent une électrovanne (21.0) comprenant :
- une entrée de vanne principale (21.1) raccordée fluidiquement à la ligne principale de prélèvement de gaz (4),
- une entrée de vanne secondaire (21.2) raccordée fluidiquement à la ligne secondaire de prélèvement de gaz (20), et
- une sortie de vanne (21.3) raccordée fluidiquement à la ligne d'analyse de gaz (5).

3. Appareil selon la revendication 1, **caractérisé en ce que** les moyens de pilotage (8) sont configurés pour contrôler les moyens à vanne (21), en particulier l'électrovanne (21.0), pour mettre la ligne d'analyse de gaz (5) en communication fluidique avec :
- soit la ligne principale de prélèvement de gaz (4) pour alimenter la ligne d'analyse de gaz (5) avec du gaz provenant du circuit patient (2),
- soit la ligne secondaire de prélèvement de gaz (20) pour alimenter la ligne d'analyse de gaz (5) avec de l'air ambiant provenant de l'atmosphère , i.e. l'air dans le local et entourant l'appareil.

4. Appareil selon la revendication 1, **caractérisé en ce que** les moyens d'aspiration de gaz (9) comprennent une pompe aspirante comprenant une entrée de gaz reliée fluidiquement à la ligne d'analyse de gaz (5) et une sortie de gaz reliée fluidiquement à l'atmosphère, de préférence via un orifice de sortie (10).

5. Appareil selon l'une des revendications 1 ou 4, **caractérisé en ce que** les moyens de pilotage (8) sont configurés pour piloter les moyens d'aspiration de gaz (7) pour aspirer du gaz, de préférence à un débit d'aspiration donné compris entre 50 et 350 mL/min.

6. Appareil selon la revendication 1, **caractérisé en ce que** les moyens de mesure de concentration (6) comprennent un capteur de NO (6.1) et/ou un capteur de NO₂ (6.2).

7. Appareil selon la revendication 1, **caractérisé en ce que** :
- l'écran tactile (9) est configuré pour afficher une touche d'activation (91), et une touche de confirmation de choix (94), actionnables par appui de l'utilisateur et
- les moyens de pilotage (8) sont configurés pour agir sur les moyens à vanne (21), en réponse à un appui par l'utilisateur sur la touche d'activation (91), et sur la touche de confirmation de choix (94), pour mettre la ligne d'analyse de gaz (5) en communication fluidique avec la ligne secondaire de prélèvement de gaz (20).

8. Appareil selon la revendication 1, **caractérisé en ce que** les moyens de pilotage (8) sont configurés pour agir automatiquement à une fréquence d'activation donnée sur les moyens à vanne (21) pour mettre la ligne d'analyse de gaz (5) en communication fluidique avec la ligne secondaire de prélèvement de gaz (20) de manière à prélever de l'air ambiant et de le soumettre aux moyens de mesure de concentration (6) afin de déterminer la teneur en NO et/ou la teneur en NO₂ de l'air ambiant prélevé.

9. Appareil selon la revendication 8, **caractérisé en ce que** la fréquence d'activation mémorisée est de plusieurs fois par jour.

10. Appareil selon la revendication 9, **caractérisé en ce que** la fréquence d'activation mémorisée est d'une à plusieurs fois par heure.

11. Appareil selon l'une des revendications 1 ou 7, **caractérisé en ce que** l'écran tactile (9) est configuré pour afficher (95) la teneur en NO et/ou la teneur en NO₂ déterminées dans l'air ambiant.

12. Appareil selon l'une des revendications 1 ou 8, **caractérisé en ce que** les moyens de pilotage (8) sont configurés pour déterminer les teneurs en NO et/ou en NO₂ dans l'air ambiant en traitant les mesures opérées par les moyens de mesure de concentration (6), après un délai de latence donné.

13. Appareil selon la revendication 12, **caractérisé en ce que** le délai de latence est compris entre de 10 à 120 secondes.

14. Appareil selon la revendication 8, **caractérisé en ce que** la fréquence d'activation est mémorisée sous forme d'un calendrier journalier.

15. Installation d'administration de gaz (100) à un patient (P) comprenant :
l'appareil de délivrance de NO (1) selon l'une des revendications 1 à 14 ; un ventilateur médical (3) ; et le circuit patient (2) auquel sont reliés fluidiquement l'appareil de délivrance de NO (1) et le ventilateur médical (3), et dans laquelle le circuit patient (2) est raccordé fluidiquement aux moyens à vanne (20), via la ligne principale de prélèvement de gaz (4).
